# EUROPEAN PATENT SPECIFICATION

(11) **EP 3 359 132 B1**
(45) Date of publication and mention of the grant of the patent: **16.08.2023**
(21) Application number: 16778788.6
(22) Date of filing: 06.10.2016
(51) Int. Cl.: A61K 9/00, A61K 9/08, A61K 47/40, A61K 35/761

(54) **METHODS FOR PREVENTING PLASTIC-INDUCED DEGRADATION OF BIOLOGICALS**
VERFAHREN ZUR KONSERVIERUNG VON BIOLOGISCHEN PRODUKTEN IN KUNSTSTOFFBEHÄLTERN
PROCÉDÉS DE CONSERVATION DE PRODUITS BIOLOGIQUES DANS DES RÉCIPIENTS EN PLASTIQUE

(30) Priority: 06.10.2015 EP 15188486
(43) Date of publication of application: 15.08.2018
(73) Proprietor: Janssen Vaccines & Prevention B.V., 2333 CN Leiden (NL)
(72) Inventor: ADRIAANSEN, Janik, 2333 CN Leiden (NL); HESSELINK, Renske, Willemijn, 2333 CN Leiden (NL)
(74) Representative: Beslier, Victor
(86) International application number: PCT/EP2016/073838
(87) International publication number: WO 2017/060329

(56) References cited:
- WO-A1-2011/098837
- WO-A1-2015/040002
- WO-A2-2012/110971
- CN-A- 104 027 815
- Julian Kissmann ET AL: "H1N1 influenza virus-like particles: Physical degradation pathways and identification of stabilizers", Journal of Pharmaceutical Sciences, vol. 100, no. 2, 1 February 2011 (2011-02-01), pages 634-645, XP055012792, ISSN: 0022-3549, DOI: 10.1002/jps.22304

## Description

### FIELD OF THE INVENTION

The present invention relates to methods for preventing surface-induced degradation of biologicals in bioprocess bags. In particular, it relates to the use of β-cyclodextrins for preventing deterioration of viruses in solutions contained in bioprocess bags. The methods of the present invention improve the compatibility of said viruses with plastic bioprocess bags by preserving quantity, structure, potency and quality of the contained viruses.

### BACKGROUND OF THE INVENTION

With a clear trend in pharmaceutical industry towards disposable systems, an ongoing challenge in the field of viruses is to generate methods for manufacturing and bulk storage of liquid compositions that contain said viruses. Especially wherein said viruses are stabilized for longer periods of time within a realistic storage temperature range for pharmaceutical products, such as from about 2°C to about 8°C. For practical and logistical reasons, bioprocess bags, which are plastic containers, are often used to store viruses. Said large structures often deteriorate inside these plastic containers, as a consequence of mechanisms that are largely unknown.

Biological activity of a virus depends upon the conformational integrity of at least a core sequence of amino acids. Unlike traditional organic and inorganic small molecules, viruses are highly complex biological structures and minor chemical or physical stressors can contribute to the degradation of the virus. Compatible primary packaging for bulk storage is of crucial importance to ensure a reasonable shelf-life, but given the nature of the disposable storage containers used in the industry, this poses particular challenges. Viruses contained in a bioprocess bag tend to lose potency as a result of surface-induced degradation.

WO2015/040002 describes adenovirus formulations comprising hydroxypropyl-β-cyclodextrin and use thereof in glass containers.

WO2011/098837 describes methods for stabilizing a modified vaccinia Ankara virus or the adenovirus AdHu5 by forming a liquid mixture of the virus with a glass-forming excipient which comprises trehalose and sucrose, applying said liquid mixture to a support and drying said liquid mixture. It has been shown that AdHu5 lost activity during the drying process.

CN104027815 describes a tanshinone inclusion fluid comprising beta-cyclodextrin and tanshinone.

WO2012/110971 discloses the use of additives as cyclodextrins to retard sorption of paraben preservatives present in a liquid formulation to the contacting plastic material or thermoplastic solid.

Accordingly, there is a need in the art to find methods for improving the compatibility of viruses with bioprocess bags that are used for manufacturing and storage of viruses. In particular there is a need for methods that protect viruses contained in bioprocess bags, from surface-induced degradation and therewith improve shelf life of said viruses during processing and storage in bulk.

### SUMMARY OF THE INVENTION

We have found and describe herein, methods for protecting a virus contained in a bioprocess bag from surface-induced degradation. These methods improve the compatibility of viruses with plastic bioprocess bags by preserving quantity, structure, potency and quality of the virus as compared to previously disclosed methods. Remarkably, the addition of a β-cyclodextrin to any solution comprising viruses, contained in a bioprocess bag, resulted in an outstanding preservation of structure and potency of said viruses, therewith unexpectedly improving the overall compatibility of said viruses with the plastic surface of the bulk storage container as compared to the same solution without the β-cyclodextrin.

The present invention relates to a method for protecting a virus from surface-induced degradation wherein said virus is contained in a solution in a bag made of a plastic wherein said method comprises the step of adding a β-cyclodextrin to said solution at a concentration between 1% (w/w) to 30% (w/w) wherein said virus is an adenovirus, said β-cyclodextrin is 2-hydroxypropyl-β- cyclodextrin, and the fluid contact layer of said bag is Ethylene Vinyl Acetate.

Also disclosed is wherein said plastic includes a gas barrier. Preferably said gas barrier is made from Ethyl Vinyl Alcohol. Also disclosed is wherein said bag is a bioprocess bag.

It was demonstrated herein, for the first time, that the addition of a β-cyclodextrin can provide protection against surface-induced degradation of a virus when stored in a bioprocess bag. The present invention also relates to the use of a β-cyclodextrin for protecting a virus from surface-induced degradation wherein said virus is contained in a solution which is contained in abag made of a plastic and wherein said virus is an adenovirus, said β-cyclodextrin is 2-hydroxypropyl-β- cyclodextrin, and said plastic bag is made of Ethylene Vinyl Acetate.

Also disclosed is wherein said plastic includes a gas barrier Also disclosed is wherein said gas barrier is Ethyl Vinyl Alcohol.

### DESCRIPTION OF THE FIGURES

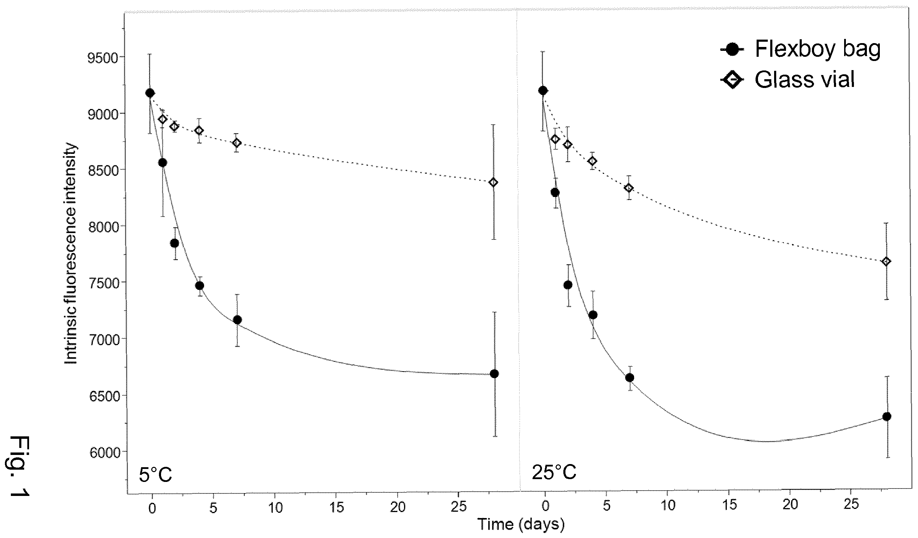

Figure 1: Compatibility of Ad26 in formulation 1 with plastic Flexboy bags (closed circles and solid lines) or glass vials (open diamonds and dotted lines), when stored at 5°C (left) or 25°C (right) for up to four weeks, as monitored by intrinsic protein fluorescence. Mean values and standard deviations are shown, with lines indicating the trends.

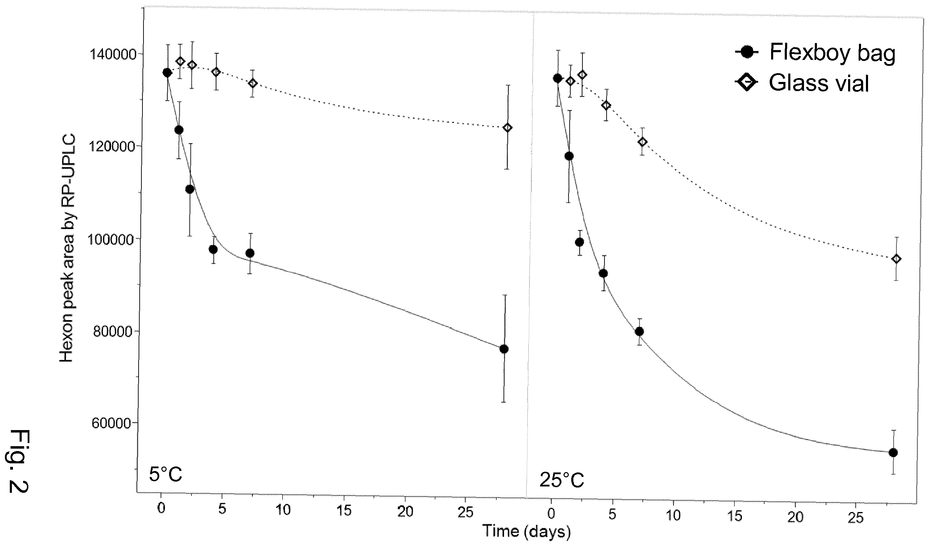

Figure 2: Compatibility of Ad26 in formulation 1 with plastic Flexboy bags (closed circles and solid lines) or glass vials (open diamonds and dotted lines), when stored at 5°C (left) or 25°C (right) for up to four weeks, as monitored by hexon content by RP-UPLC. Mean values and standard deviations are shown, with lines indicating the trends.

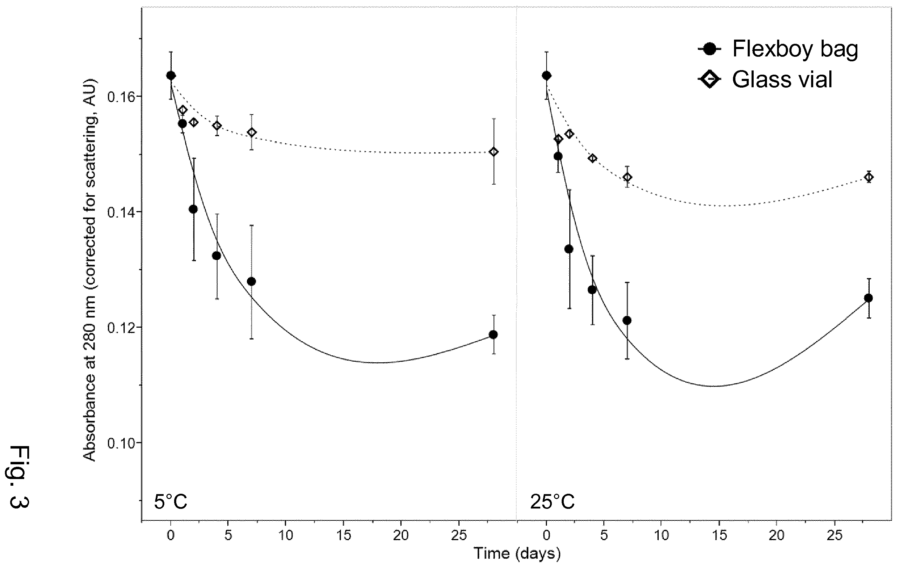

Figure 3: Compatibility of Ad26 in formulation 1 with plastic Flexboy bags (closed circles and solid lines) or glass vials (open diamonds and dotted lines), when stored at 5°C (left) or 25°C (right) for up to four weeks, as monitored by protein concentration by absorbance at 280 nm. Mean values and standard deviations are shown, with lines indicating the trends.

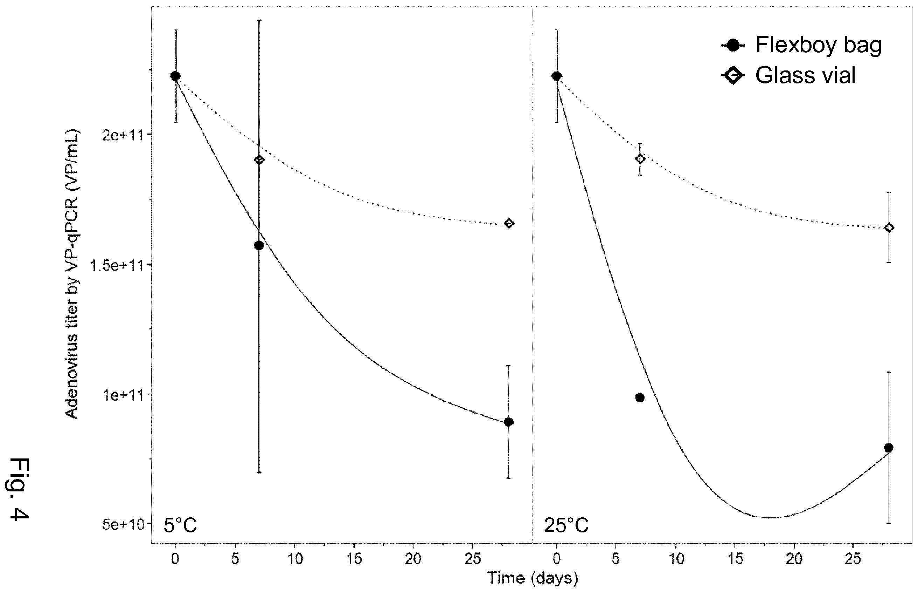

Figure 4: Compatibility of Ad26 in formulation 1 with plastic Flexboy bags (closed circles and solid lines) or glass vials (open diamonds and dotted lines), when stored at 5°C (left) or 25°C (right) for up to four weeks, as monitored by adenovirus titer by vp-QPCR. Mean values and standard deviations are shown, with lines indicating the trends.

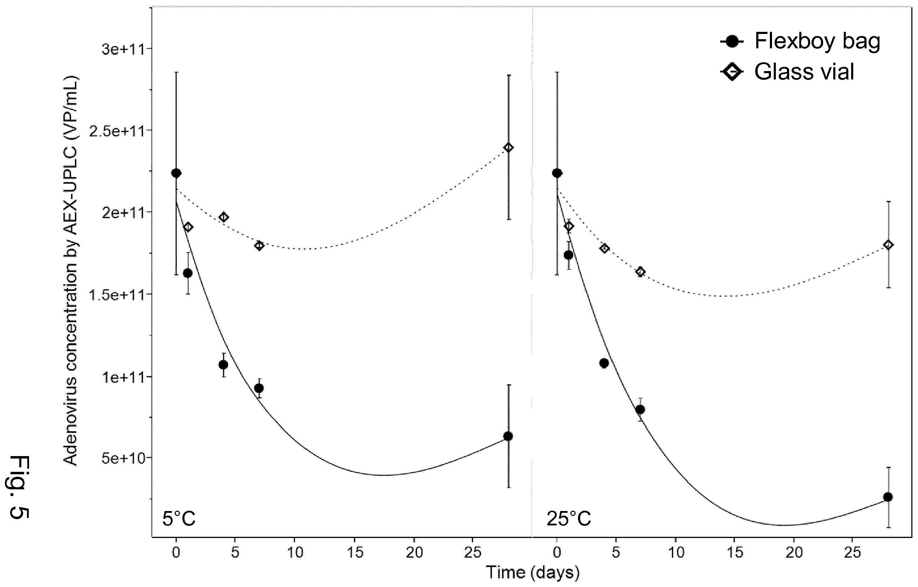

Figure 5: Compatibility of Ad26 in formulation 1 with plastic Flexboy bags (closed circles and solid lines) or glass vials (open diamonds and dotted lines), when stored at 5°C (left) or 25°C (right) for up to four weeks, as monitored by adenovirus concentration by AEX-UPLC. Mean values and standard deviations are shown, with lines indicating the trends.

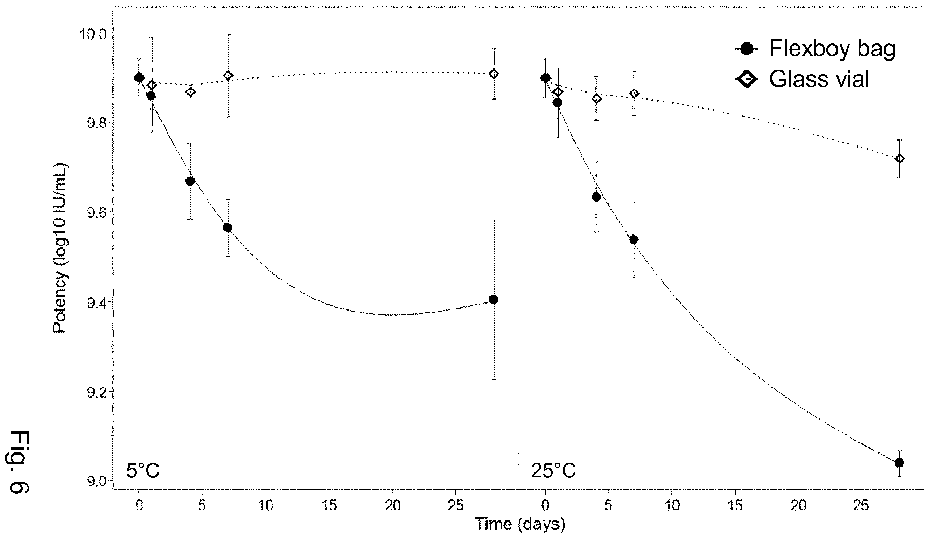

Figure 6: Compatibility of Ad26 in formulation 1 with plastic Flexboy bags (closed circles and solid lines) or glass vials (open diamonds and dotted lines), when stored at 5°C (left) or 25°C (right) for up to four weeks, as monitored by potency by QPA. Mean values and standard deviations are shown, with lines indicating the trends.

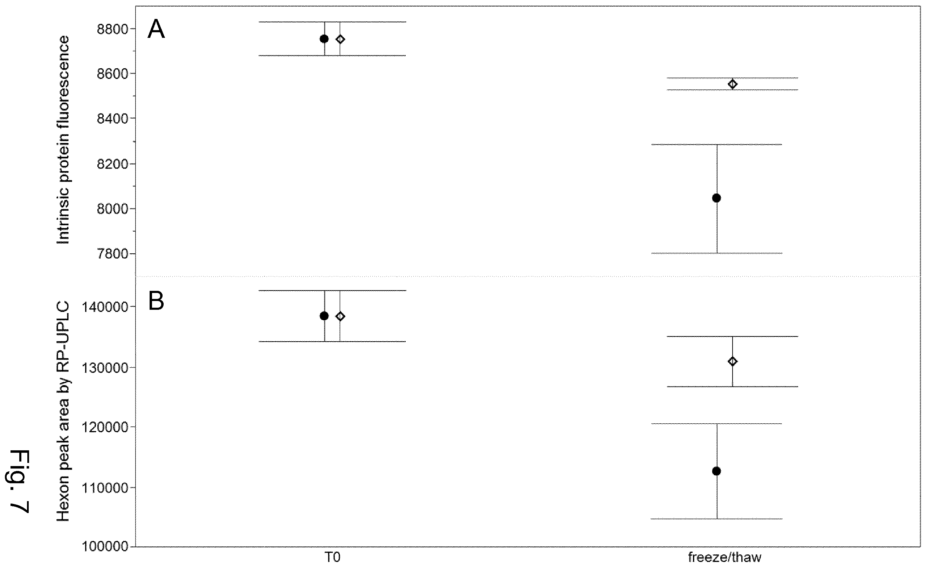

Figure 7: Compatibility of Ad26 in formulation 1 with plastic Flexboy bags (closed circles) or glass vials (open diamonds) during one freeze/thaw cycle (room temperature to -70°C and back, at 0.032°C/min), as monitored by intrinsic protein fluorescence (A) and hexon content by RP-UPLC (B). Mean values and standard deviations are shown.

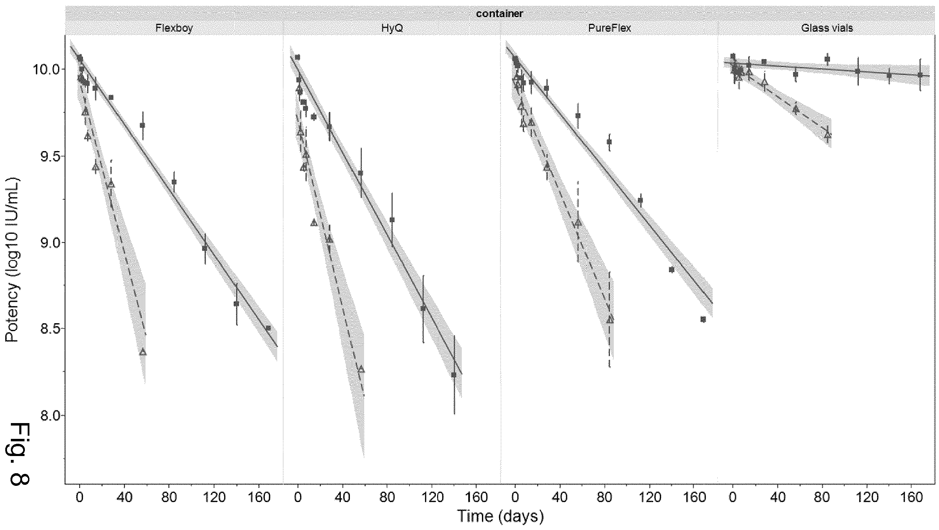

Figure 8: Compatibility of Ad26 in formulation 1 with (from left to right) Flexboy^{®} bioprocessing bags (Sartorius Stedim Biotech GmbH), HyQ Cx5-14 bioprocessing bags (Thermo Fisher), PureFlex Mobius bioprocessing bags (Millipore), or glass vials (Nuova Ompi). Formulations were stored at 5°C (closed squares) or 25°C (open triangles) for up to 24 weeks, and compatibility was monitored by potency by QPA. Mean values and standard deviations are shown, with linear fits to the data, with 95% confidence intervals shaded grey.

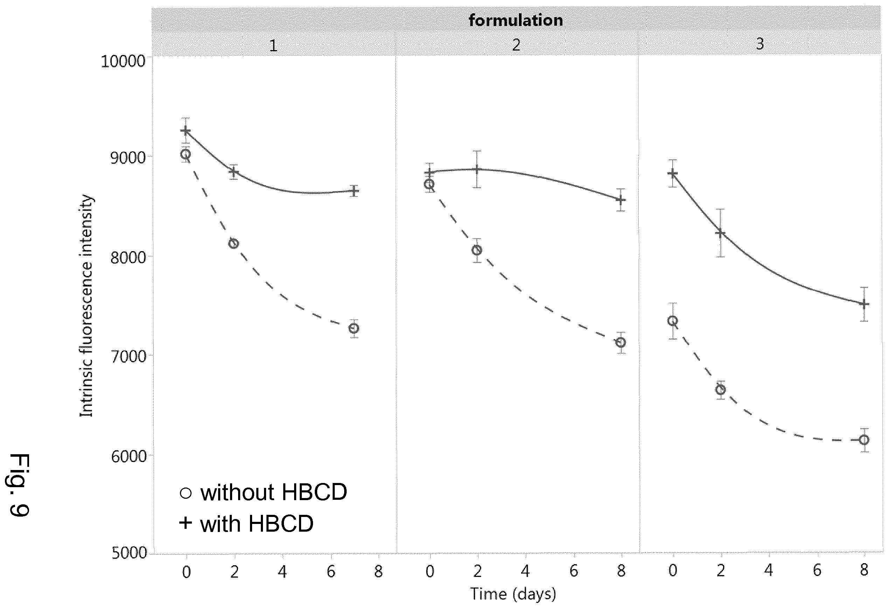

Figure 9: Compatibility of Ad26 in formulations 1, 2 and 3 (from left to right), either without HBCD (open circles and dashed lines) or supplemented with 5% (w/w) HBCD (crosses and solid lines), with plastic Flexboy bags during storage at 25°C for up to one week, as monitored by intrinsic protein fluorescence. Mean values and standard deviations are shown, with lines indicating the trends.

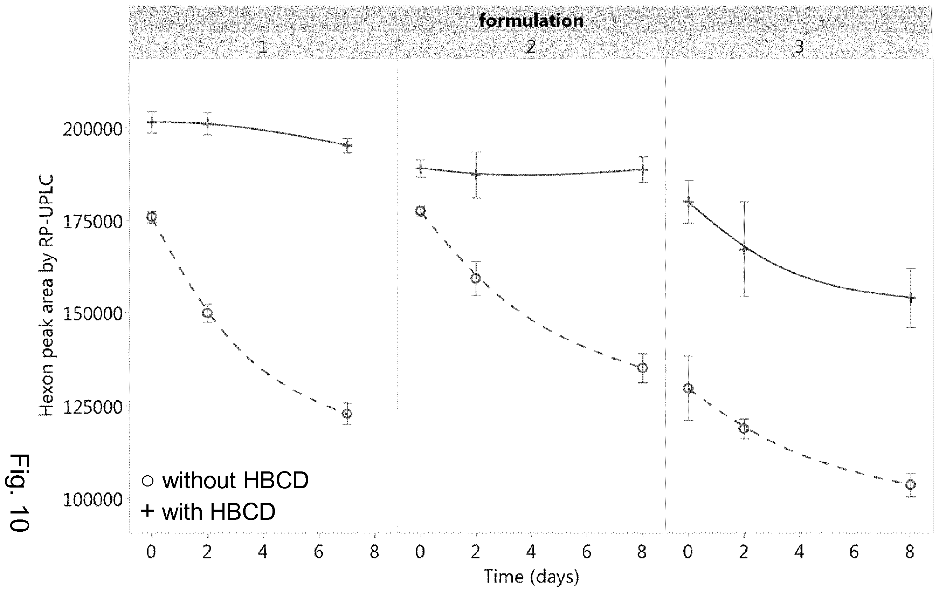

Figure 10: Compatibility of Ad26 in formulations 1, 2 and 3 (from left to right), either without HBCD (open circles and dashed lines) or supplemented with 5% (w/w) HBCD (crosses and solid lines), with bioprocess bags during storage at 25°C for up to one week, as monitored by hexon content by RP-UPLC. Mean values and standard deviations are shown, with lines indicating the trends.

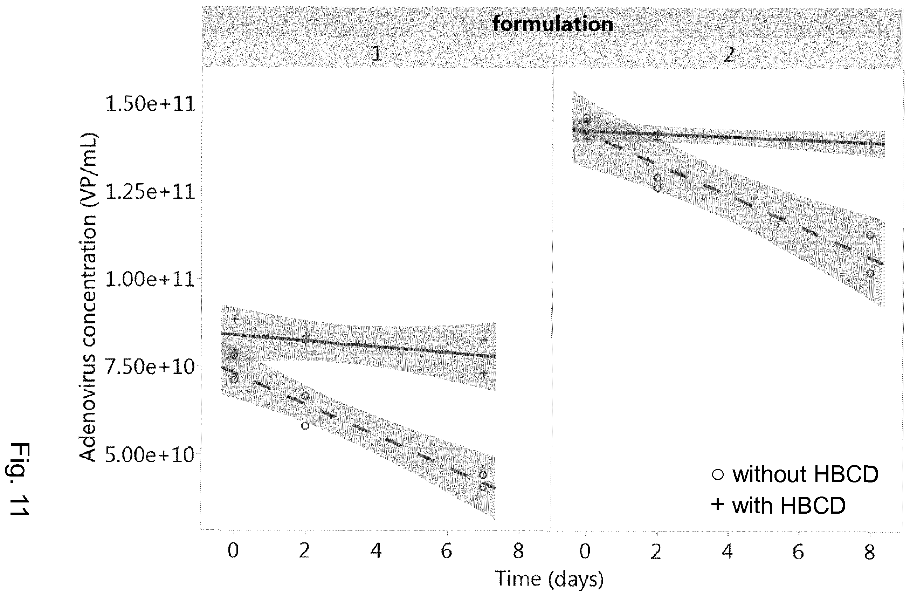

Figure 11: Compatibility of Ad26 in formulations 1 and 2 (from left to right), either without HBCD (open circles and dashed line) or supplemented with 5% (w/w) HBCD (crosses and solid line), with bioprocess bags during storage at 25°C for up to one week, as monitored by Adenovirus particle concentration by CE. Lines show linear fits to the data, with 95% confidence intervals shaded grey. Formulation 3 without HBCD was degraded to such an extent after contact with the bioprocess bag that no virus particle concentration could be determined.

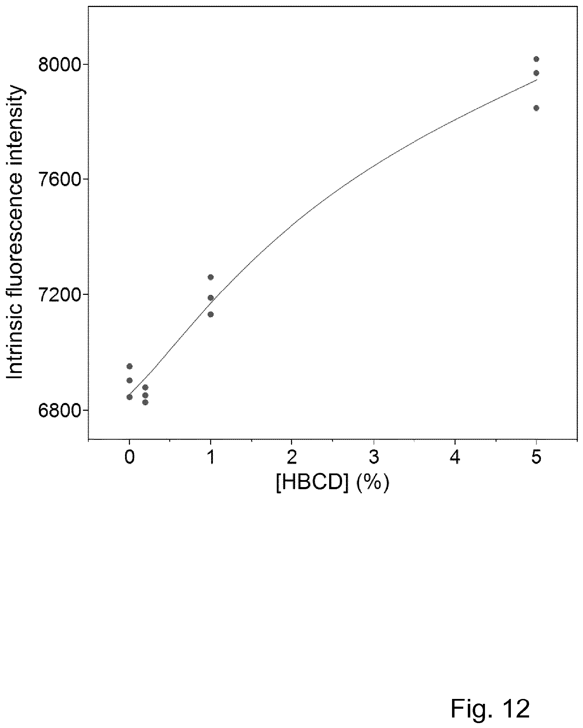

Figure 12: Compatibility of Ad26 in formulation 2 supplemented with 0, 0.2, 1 or 5% (w/w) HBCD with plastic Flexboy bags after 8 days storage at 25°C, as monitored by intrinsic protein fluorescence. Individual data points are shown, with a line indicating the trend.

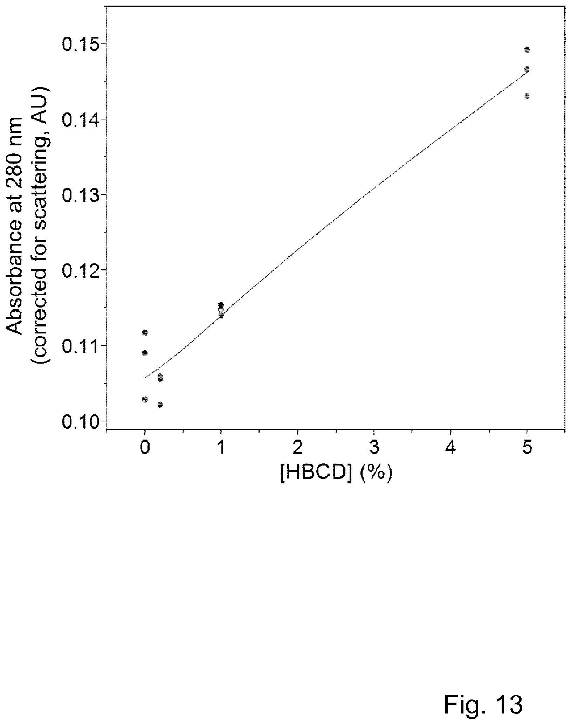

Figure 13: Compatibility of Ad26 in formulation 2 supplemented with 0, 0.2, 1 or 5% (w/w) HBCD with plastic Flexboy bags after 8 days storage at 25°C, as monitored by protein concentration by absorbance at 280 nm. Individual data points are shown, with a line indicating the trend.

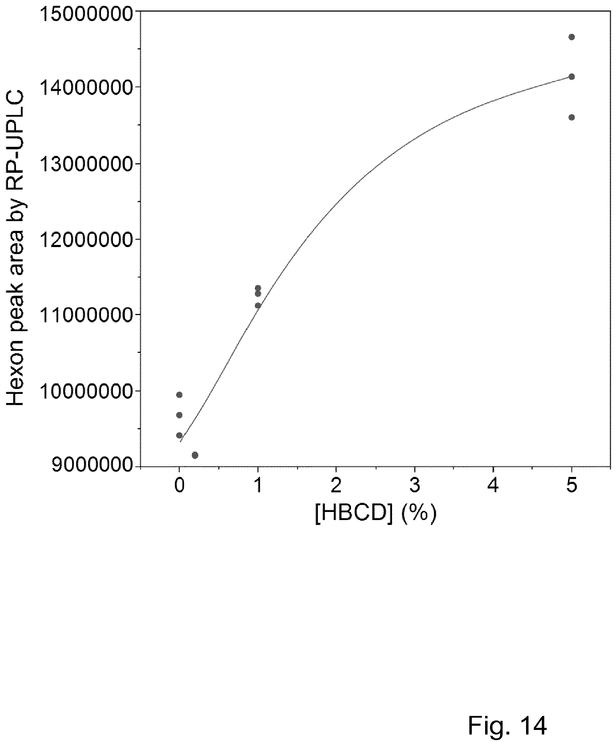

Figure 14: Compatibility of Ad26 in formulation 2 supplemented with 0, 0.2, 1 or 5% (w/w) HBCD with plastic Flexboy bags after 8 days storage at 25°C, as monitored by hexon content by RP-UPLC. Individual data points are shown, with a line indicating the trend.

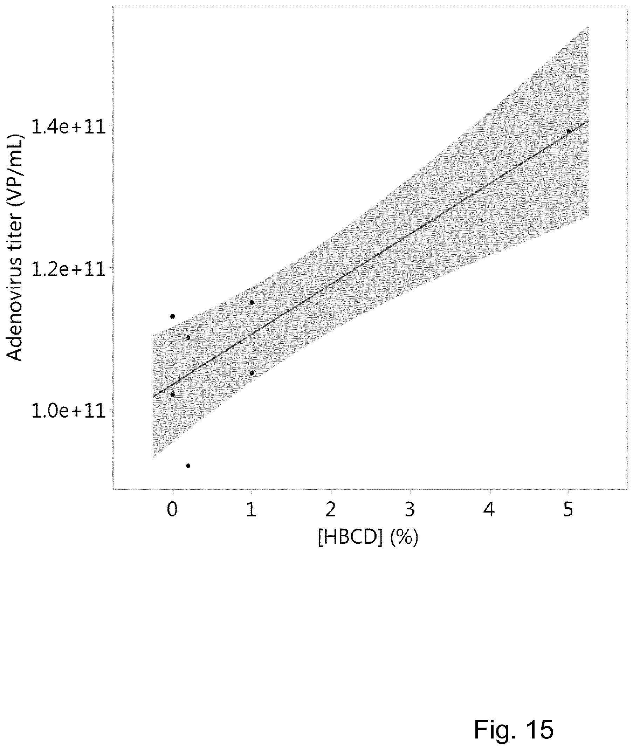

Figure 15: Compatibility of Ad26 in formulation 2 supplemented with 0, 0.2, 1 or 5% (w/w) HBCD with plastic Flexboy bags after 8 days storage at 25°C, as monitored by Adenovirus particle concentration by CE. The line shows a linear fit to the data, with the 95% confidence interval shaded grey.

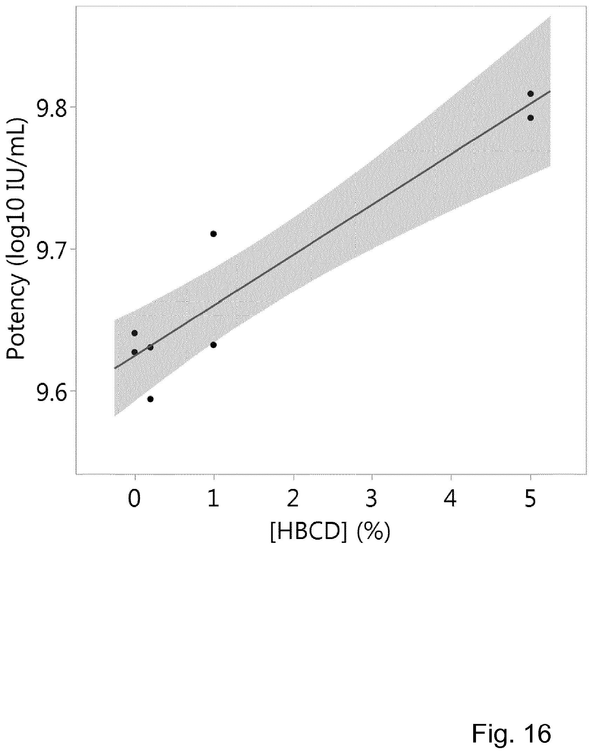

Figure 16: Compatibility of Ad26 in formulation 2 supplemented with 0, 0.2, 1 or 5% (w/w) HBCD with plastic Flexboy bags after 8 days storage at 25°C, as monitored by potency by QPA. The line shows a linear fit to the data, with the 95% confidence interval shaded grey.

### DETAILED DESCRIPTION OF THE INVENTION

As mentioned previously, there is a need to find methods for protecting a virus contained in a bioprocess bag from surface-induced degradation, thereby improving the compatibility of viruses with process and bulk storage materials by preserving quantity and potency of the contained viruses. It was shown herein that the viruses quickly degrade in bioprocess bags and that the addition of a β-cyclodextrin like 2-hydroxypropyl- β-cyclodextrin (all molar substitutions, HBCD) to a virus in solution stored in a bioprocess bag surprisingly prevents the plastic surface-induced degradation of said virus.

The invention is defined in the appended claims and any other aspects or embodiments set forth herein are for information only.

We have found and describe herein, methods for preserving viruses in bioprocess bags. In particular, these methods protect viruses from surface-induced degradation when said viruses are contained in a solution in a bag comprised of a plastic. These methods improve the biological compatibility with plastics by preserving quantity and potency and quality of the virus as compared to previously disclosed methods regardless of the formulation matrix used. Examples of the viruses are, but not limited to, (active) viruses, vaccines, non-enveloped viruses, enveloped viruses, virus-like particles, recombinant viruses, and inactivated and attenuated viruses.

In a preferred embodiment of the present invention, the virus is a recombinant adenovirus. The construction and propagation of adenoviral vectors is well understood in the art and involves the use of standard molecular biological techniques, such as those described in, for example, Sambrook et al., Molecular Cloning, a Laboratory Manual, 2d ed., Cold Spring Harbor Press, Cold Spring Harbor, N.Y. (1989), Watson et al., Recombinant DNA, 2d ed., Scientific American Books (1992), US patent 6,492,169 or in WO 03/104467 U.S. Pat. Nos. 5,559,099, 5,837,511, 5,846,782, 5,851,806, 5,994,106, 5,994,128, 5,965,541, 5,981,225, 6,040,174, WO 96/26281, WO 00/03029, and Thomas Shenk, "Adenoviridae and their Replication". In certain embodiments of the present invention, serotypes of human adenovirus include any one of serotypes 2, 4, 5, 7, 11, 26, 34, 35, 36, 48, 49 or 50 or any hybrid or mutated adenovirus serotypes. In a preferred embodiment of the present invention the recombinant adenovirus is from human adenovirus serotype 5, 26 or 35. In further embodiments, the adenovirus of the invention is a simian adenovirus, preferably a chimpanzee or gorilla adenovirus. These adenoviruses generally have a low seroprevalence and/or low pre-existing neutralizing antibody titers in the human population. In further embodiments, the adenovirus of the invention further comprises heterologous nucleic acid. Suitable heterologous nucleic acid is well known to the skilled person, and for instance may include transgene open reading frames, for instance open reading frames coding for polypeptides against which an immune response is desired when the vector is used for vaccination purposes, e.g. transgenes suitable to generate an immune response against malaria (see e.g. WO 2004/055187), HIV, Ebola, RSV, HPV, Zikavirus, HSV, Tuberculosis (see e.g. WO 2006/053871), certain viruses, etc, all well known to the skilled person. In fact, the nature of the transgene is not critical to the current invention, it may be any heterologous nucleic acid sequence, and hence needs no further elaboration here.

A few references disclose the use of cyclodextrins as excipient for the formulation of adenoviruses. WO029024 discloses hydroxypropyl-β-cyclodextrin as part of a large list of possible lyoprotectants used for preparing a freeze dried formulation. WO029024 relates to a freeze dried composition as opposed to a liquid composition as disclosed in the present invention. The advantage of a liquid formulation is that it is less expensive, and the handling before administration is less time consuming and less prone to clinical dosing or reconstitution mistakes. Furthermore, scale up of lyophilization processes can be a cumbersome endeavor. Renteria et al. identifies HBCD as one of the additives used to promote the stability of certain proteins and to avoid aggregation during nasal administration. Renteria et al. discloses the use of hydroxypropyl-β-cyclodextrin in the context of a formulation appropriate for nasal administration enhancing mucosal uptake. WO2015/04002 discloses complex multi-component virus-containing compositions containing HBCD as one of the components. The virus-containing compositions which comprise HBCD and which are exemplified in WO2015/04002, are all contained in glass vials. WO2015/04002 is silent about the incompatibility of Adenoviruses with plastic bioprocess bags.

HBCD is typically used to increase the solubility of small molecules (e.g. diclofenac or ibuprofen). This has to do with its unusual structure. The HBCD molecule is a torus shaped ring with a polar hydrophilic outside and an apolar hydrophobic cavity due to the spatial distribution of its external hydrophilic properties. The secondary OH groups are on the opposite edge. These hydroxyl groups give HBCD its external hydrophilic properties. The inside of the HBCD ring is small in diameter (much smaller than a viral particle) and fits only small molecules. It is composed of a surface of hydrophobic hydrogens as well as glycosidic ether-like oxygen. As a consequence of this particular structure, HBCD is used to encapsulate or entrap very small molecules solubilizing them to form the so-called inclusion compounds. These structural features of HBCD do not render HBCD an obvious choice for use in solving incompatibility problems in bioprocess bags.

The term "compatibility" refers to the ability of two materials or components to exist in contact with or next to each other, without negatively influencing attributes such as quantity, structure, potency or quality of one or both of the components. As used herein it refers to the relative resistance to degradation of virus particles in contact with or next to the surface of a bioprocess bag or other packaging material.

The term "potency" as used herein refers to a measure of adenovirus activity expressed in terms of infectious units measured in a cell-based potency assay, which is described hereunder.

The term "by-product" includes undesired products, which detract or diminish the proportion of therapeutic/prophylactic adenovirus in a given formulation. Typical by-products include aggregates of the adenovirus and fragments of the adenovirus, resulting from e.g. protein denaturation, deamidation, hydrolysis or combinations thereof. Typically, aggregates are complexes that have a molecular weight greater than the isolated virus particle.

A method that protects a virus from surface-induced degradation as used herein and which thereby improves the compatibility with plastics, is a method that preserves the physical and/or chemical integrity and/or biological activity of the virus upon storage in a bioprocess bag. This can be assessed by determining different characteristics such as the potency, and/or other quality aspects of the virus in the bioprocess bags over a period of time and under certain storage conditions. These characteristics of said virus can be measured at elevated temperatures (predictive for real-time temperatures) or under other stress conditions, for instance viruses can be subjected to incubation at 25°C in order to study the effects of different conditions maximizing biological shelf-life. Said characteristics which determine the degradation of viruses may be determined by at least one of the methods selected from the group consisting of visual inspection, virus particle quantitative polymerase chain reaction (vp-QPCR), QPCR-based Potency Assay (QPA), Reverse Phase High Performance Liquid Chromotography (RP-UPLC), AEX-UPLC, Intrinsic Fluorescence and protein concentration by absorbance at 280 nm.

### Virus particle quantitative polymerase chain reaction (vp-QPCR)

The vp-QPCR was developed for the quantification of adenovirus particles using primers that target a 100 bp region of the CMV promoter of the transgene cassette present within the adenovirus vector. Briefly, this QPCR method relies on the exonuclease activity of Taq polymerase, which results in degradation of a specific fluorescent probe annealed in the middle of the 100 bp amplicon. The probe is covalently linked to a light emitter and a quencher, and its degradation frees the emitter from the quencher with a consequent fluorescence emission proportional to the amount of template. Quantitative values are obtained from the threshold cycle (Ct), the cycle at which an increase in fluorescence signal exceeds a threshold value. The threshold for detection of DNA-based fluorescence is set slightly above background. The number of cycles at which the fluorescence exceeds the threshold is called the threshold cycle (Ct) or, according to the MIQE guidelines, quantification cycle (Cq) (Bustin et al, 2009). During the exponential amplification phase, the target DNA sequence doubles every cycle. For example, a DNA sample of which the Ct precedes that of another sample by 3 cycles contained 2³ = 8 times more template. Consequently, a higher Ct value represents a lower amount of target DNA and a lower Ct value represents a high availability of target DNA. Absolute quantification can be performed by comparing a standard curve generated by a serial dilution of a stock adenovirus of which the concentration has been determined by the optical density at 260 nm (OD₂₆₀). The Ct value of the test material is plotted against the Ct values of the standard curve, which generates an accurate and precise number of vector particles.

When used as readout after incubation on E1 competent cells (QPA, see below), more degraded samples will lead to higher delta (t=0 subtracted) Ct values and more stabilizing formulations will lead to lower Ct values.

### QPCR-based Potency Assay (QPA)

To quantify adenovirus potency, the QPA combines QPCR with a tissue culture-based infectivity assay. The assay is based on the experimental observation that the appearance of newly synthesized viral DNA is very rapid after inoculation of a cell-monolayer, and is proportional to the virus input concentration over a large range of multiplicity of infection (MOI). Dilutions of samples (non-endpoint diluted) are inoculated onto HEK293 cell monolayers in a 96-well plate. The infection is allowed to proceed for 3 hours at 35°C. Wells are aspirated and replenished with medium that does not contain adenoviruses. Plates are incubated for an additional 42 hours prior to cell lysis by means of Triton X-100 solution and a single freeze/ thaw step in order to release adenovirus DNA. A QPCR is performed on diluted cell lysates according to the method described above. The infectivity titer is calculated by comparison to a standard curve generated by the Ct values of a sample of known infectivity, which is determined by endpoint titration. Alternatively, the delta potency can be expressed directly as Ct values since the infectivity titer, or potency, is directly correlated to the Ct values. Especially in comparing relative differences in potency between formulations, this is a quick and reliable method.

### Reversed-Phase Ultrahigh-Performance Liquid Chromatography (RP-UPLC)

In order to determine several quality attributes of an adenovirus, such as presence and (relative) quantity of adenoviral and other proteins, one can analyze adenoviral protein profiles by Reversed-Phase Ultrahigh-Performance Liquid Chromatography (RP-UPLC). UPLC separates components of a mixture by using a variety of chemical interactions between the sample, the mobile phase (a buffer or solvent) and the stationary phase (a chromatographic packing material in a column). A high-pressure pump moves the mobile phase through the column, while the analyte partitions between the two phases, so that its elution depends on its relative affinities for the stationary and mobile phases. A detector measures the retention times (*t*_{R}; time between sample injection and the appearance of the peak maximum) of the molecules using e.g. UV absorbance detection.

The separation mechanism of RP-UPLC is based on differences in hydrophobicity. In the adenovirus protein profiling method, the non-polar stationary phase is made up of hydrophobic alkyl C4 chains, while the mobile phase consists of a water / acetonitrile / TFA mixture with increasing hydrophobicity. Adenoviral particles dissociate into their constituent proteins, which initially interact with the stationary phase, and subsequently elute at a retention time depending on their hydrophobic surface area. The retention time and amount of each protein are monitored by UV absorbance detection at 280 nm. A typical adenoviral RP-UPLC profile consists of 10 or 14 proteins, including core protein (VII), penton base (III) and hexon (II).

### Anion-Exchange Ultrahigh-Performance Liquid Chromatography (AEX-UPLC)

In order to quantify the number of adenovirus particles, one can separate the virus particles from the matrix by Anion-Exchange Ultrahigh-Performance Liquid Chromatography (AEX-UPLC), and quantify them by UV absorbance.

UPLC separates components of a mixture by using a variety of chemical interactions between the sample, the mobile phase (a buffer or solvent) and the stationary phase (a chromatographic packing material in a column). A high-pressure pump moves the mobile phase through the column, while the analyte partitions between the two phases, so that its elution depends on its relative affinities for the stationary and mobile phases. A detector measures the retention times (*t*_{R}; time between sample injection and the appearance of the peak maximum) of the analytes using e.g. UV absorbance detection.

In the AEX-UPLC method for adenovirus particle quantification, the negatively charged particles are captured via their interaction with a positively charged column containing quaternary ammonium cations. Increasing salt concentration in the mobile phase weakens the electrostatic interactions, so that the viral particles elute at a retention time specific for their serotype, separate from matrix components and impurities. The particles are subsequently quantified by UV absorbance at 214 nm.

### Capillary zone electrophoresis (CE)

Capillary zone electrophoresis (CE) is used to determine the Adenovirus particle concentration. In this method, a sample containing virus particles moves through a small diameter capillary via electroosmotic flow. The various components in the sample have different electrophoretic mobilities, because of size, charge and frictional differences. This causes the different components to separate into bands.

Upon elution, the Adenovirus particle peak is detected and quantified by absorbance at 214 nm. The signal is converted into a concentration via a calibration standard.

### Intrinsic fluorescence assay

The adenoviral capsid proteins contain aromatic amino acids that reemit light after excitation, in particular tryptophan and to a lesser extent tyrosine and phenylalanine. The emission maximum and quantum yield of tryptophan depend strongly on the polarity of its environment. In a polar, aqueous environment (e.g. the surface of a globular protein) the quantum yield is relatively low, while in an apolar environment (e.g. the inside of an aggregate) the quantum yield increases. This feature makes tryptophan fluorescence a useful tool for studying protein conformational change, aggregation, and molecular interactions.

In the intrinsic fluorescence assay, samples are transferred in triplicate to a UV-transparent, flat-bottom microplate. The plate is covered with a UV-transparent seal. Tryptophan fluorescence is measured by a microplate reader using an excitation filter with a center wavelength of 280 nm and a bandwidth of 10 nm, and an emission filter with a center wavelength of 340 nm and a bandwidth of 10 nm. Bottom optic is used to minimize the influence of the seal and the meniscus shape.

The fluorescence intensity is known in the art to be a sensitive measure of adenovirus degradation. Either an increase or a decrease may be observed upon stress, depending on the nature of the changes occurring in the sample. Protein unfolding and capsid dissociation is expected to lead to a decrease in intrinsic fluorescence, and aggregation is expected to lead to an increase. The precision of the assay is < 5% (CV%) in the range used.

The obtained fluorescence for stressed samples should always be compared to the control samples. Since an increase or decrease after applied stress is dependent on the degradation pathway and specific for each Active Pharmaceutical Ingredient (API), it cannot be predicted. A change (higher or lower) compared to the t=0 samples is indicative of a less stable formulation. Stressed samples remaining close to the t=0 sample values are more stable.

### Protein concentration by absorbance at 280 nm

The adenoviral capsid proteins contain aromatic amino acids that absorb ultraviolet light around 280 nm, in particular tryptophan and to a lesser extent tyrosine and phenylalanine. The absorbance is linearly correlated with the number of amino acids in the optical pathlength, and thereby to the protein concentration, according to the Beer-Lambert law. Since the viral particles also scatter light, resulting in a wavelength-dependent apparent absorbance, the signal is first corrected for this scattering signal to obtain a true absorbance signal. The absorbance at 280 nm is subsequently either translated to a protein concentration via a calibration curve, or directly compared between samples as a quantitative measure of protein concentration.

A virus is compatible with a primary bulk container such as a bioprocess bag, if amongst others, it shows minimal loss (i.e. 0.3log/2 years) in terms of quantity and potency, and displays no major modifications. Additionally, no signs of aggregation, precipitation, change of color and/or clarity upon visual examination should be observed.

"About" as used in the present application means ±10%, unless stated otherwise.

"Plastic" as used in the present application means: synthetic or semi-synthetic malleable (co-)polymer of one or more organic molecules. This includes films consisting of multiple layers, such as a fluid contact layer, a gas barrier layer, and outer layers, of which at least the fluid contact layer is a plastic. Examples of plastics used in the present application are Ethylene Vinyl Acetate (including monolayer), (Ultra-Low Density) Polyethylene, Polyamide and Polyethylene.

"Bag" as used in the present application means: a bioprocess container made of plastic, which may be used during the manufacturing process of viruses, in order to hold process intermediates or (bulk) viruses, or as primary packaging. Examples of bags as used in the present application are e.g. Flexboy bags, HyQ bags, PureFlex bags, wave bags, bioreactors, etc. These bags are commonly referred as bioprocess bags. The term "bag" and "bioprocess bag" can interchangeably be used within the present application.
"polymer" as used in the present application means: a large molecule, or macromolecule, composed of many repeated subunits (monomers).
"co-polymer" as used in the present application means: two or more different monomers united together.

In a preferred embodiment of the invention, the bioprocess bag is made of a polymer. In a more preferred embodiment of the invention, said polymer is selected from the group of Ethylene Vinyl Acetate (including monolayer), (Ultra-Low Density) Polyethylene, Polyamide and Polyethylene.

"Cyclodextrin" as used in the present application means: group of cyclic oligosaccharides. Among the most commonly used forms are α-, β-, and γ-cyclodextrin, which have respectively 6, 7, and 8 glucose molecules. In accordance with the present invention, the cyclodextrins that offer protection to viruses against surface-induced degradation in bags are β-cyclodextrins. Substituted derivatives are also available, like dimethyl-β-cyclodextrin, 2-hydroxyethyl-β-cyclodextrin, 2-hydroxypropyl-β-cyclodextrin, 3-hydroxypropyl-β-cyclodextrin and trimethyl-β-cyclodextrin and 2-hydroxypropyl-β-cyclodextrin. For the latter group of molecules, "The molar substitution (MS)" as used in the present application means: the average number of hydroxypropyl groups per anhydroglucose unit.

"The degree of substitution (DS)" as used in the present application means: the number of hydroxypropyl groups per molecule of cyclodextrin.
"gas barrier" as used in the present application means: a layer impermeable to gas in between two layers of monomers, such as ethyl vinyl alcohol.
"w/w" as used in the present application means: "weight for weight" or "weight by weight", the proportion of a particular substance within a mixture, as measured by weight or mass.

The present invention relates to methods for protecting viruses from surface-induced degradation in bioprocess bags. These methods improve the compatibility with plastics by preserving quantity and potency (infectivity) and quality of the viruses as compared to previously disclosed methods. Remarkably, the addition of a β-cyclodextrin to a solution comprising a virus, contained in a bioprocess bag resulted in an outstanding preservation of quantity, potency (infectivity) and quality of said virus, therewith improving the overall compatibility of said virus with bioprocess bags as compared to the solution without the β-cyclodextrin.

The present invention relates to methods for preserving viruses and related pharmaceutical products in a solution, preferably for use in gene therapy and/or vaccine applications. The solutions that contain the virus are appropriate for manufacturing and storage in the 2-8°C range while also being compatible with parenteral administration. These solutions could also be stored at lower temperatures, e.g. -20°C or lower, -40°C or lower, -65°C or lower, -80°C or lower. They may also be stored at temperatures above 8°C, e.g. 25°C or even higher.

The solutions used in the present invention provide compatibility to plastic, to the viruses at varying concentrations, and may be administered to a variety of vertebrate organisms, preferably mammals and especially humans. The stabilized solutions used in the methods of the present invention are e.g. viral-based compositions, which can, for instance, be administered as a vaccine that may offer a prophylactic advantage to previously uninfected individuals and/or provide a therapeutic effect.

In the present invention the β-cyclodextrin is 2-hydroxypropyl-β-cyclodextrin.

In the present invention the concentration of the β-cyclodextrin in the virus-containing solution is ranging between 1% (v/v) and 30% (v/v), e.g. between 1.5% (v/v) and 25% (v/v), e.g. between 2% (v/v) and 20%, e.g. between 2.5% (v/v) and 20% (v/v), e.g. between 3% (v/v) and 15% (v/v), e.g. between 3.5% (v/v) and 10% (v/v), e.g. 5% (v/v).

The following examples are provided to illustrate the present invention without, however, limiting the same hereto.

### EXAMPLES

### Example 1

### Experimental design and methodology

The compatibility with glass and plastic of an Adenoviral preparation comprising an Ad26 adenovirus (as described in [1]) was tested. Ad26 adenoviruses were filled in Flexboy^{®} bioprocessing bags (Sartorius Stedim Biotech GmbH) and in glass vials (Nuova Ompi), at a concentration of 2.4×10¹¹ VP/mL in the standard formulation 1 (see Table 1). The 50 mL Flexboy^{®} bioprocessing bags were filled with 20 mL of the formulation, while the 3 mL vials were filled with 1.5 mL (in both cases in duplicate per condition and time point). Control samples were taken from the formulation at T=0 before filling the bags and vials.

To evaluate the compatibility of the Adenovirus formulation with plastic and glass surfaces, the bags and vials were incubated at 5 ± 3°C or at 25 ± 2°C for up to four weeks, with time points at 1, 2, 4, 7 and 28 days. At each time point, samples were removed from the bags and vials and stored at ≤ -65°C until sample analysis.

The compatibility of the Adenovirus formulation with plastic as compared to glass and as compared to the T=0 control samples, was analyzed by the following methods, as described above: intrinsic protein fluorescence intensity; hexon protein content by RP-UPLC; total protein content by absorbance at 280 nm; Adenovirus particle concentration by vp-QPCR; Adenovirus particle concentration by AEX-UPLC; and potency (infectivity) by QPCR-based Potency Assay (QPA). The analyses were performed on all bags and vials at all time points, except for the vp-QPCR analysis, which was performed for a selection of samples only.

In order to further evaluate the unexpected finding that the standard formulation 1 was incompatible with the bioprocess bag (see results section), additional contact materials and time points were studied. Ad26 adenoviruses in formulation 1 at a concentration of 3.0×10¹¹ VP/mL was incubated at 5 ± 3°C or at 25 ± 2°C for up to six months, in Flexboy^{®} bioprocessing bags (Sartorius Stedim Biotech GmbH), in HyQ Cx5-14 bioprocessing bags (Thermo Fisher), or in PureFlex Mobius bioprocessing bags (Millipore); in all cases, 25 mL of the formulation was filled into a 50 ml bag. Glass vials (Nuova Ompi) were included as a control.

At time points 0, 1, 2, 5, 6, 7 and 14 days, and 1, 2, 3, 4, 5 and 6 months, samples were removed from the bags and vials and analyzed for compatibility using the methods as described above.

### Results and conclusions

Figs. 1-3 show the compatibility of Ad26 adenoviruses with either glass or plastic in formulation 1. Intrinsic fluorescence shows the protein content and conformation, hexon peak area the amount of hexon protein, and absorbance at 280 nm the total protein concentration. The results show that, surprisingly, the amount of (total and viral) protein decreases when formulation 1 is in contact with a plastic surface, already after 1 day at 5 or 25°C. The decrease is strongest during the first week, during which a ca. 20 - 30% drop in protein content occurs. The degradation is only slightly mitigated at the lower temperature. The effect is clearly induced by the presence of the plastic surface, since the material does not show these changes in protein content when incubated in glass vials. This incompatibility of the viruses with plastic is a very unexpected finding, since plastic bioprocessing bags are commonly used for manufacturing and storage of proteins, viruses and other biologicals.

The virus particle concentration follows a similar trend (Fig. 4 and 5), with a decrease of almost 50% for the samples in contact with bioprocess bags, as compared to no significant decreases for the samples in glass vials. Fig. 6 shows that infectious particles are affected by the effect, so that the potency decreases concurrently with the viral proteins and particles in the samples in bioprocess bags.

A similar effect was observed when the samples were subjected to freeze/thaw stress (Fig. 7). After a single freeze/thaw cycle, the material in bags showed a clear decrease in protein content, while the sample in glass indicated that the material itself is resistant to this stress condition. Frozen storage of the virus material is thus not a solution that can prevent the surface-induced degradation.

To investigate whether the incompatibility is specific for the type of bioprocess bag used in Figs. 1 - 7, several other types of plastic bags were tested for compatibility as well. Fig. 8 shows the potency, expressed as concentration of infectious particles, of a virus preparation stored in three types of bioprocess bags, as compared to glass vials. The potency decreases strongly, at both 5 and 25°C, when the virus material is in contact with any of the plastics. The virus material stored in glass vials, on the other hand, shows good compatibility, and degrades at a much slower rate than in the bioprocess bags at both temperatures. This shows that the incompatibility of the viruses with plastics is independent of the type of plastic, and that the problem is not easily mitigated by changing to a different type of bag.

The results demonstrate that the standard formulation 1 degrades rapidly upon contact with a plastic surface, and that this incompatibility cannot easily be mitigated by e.g. a lower temperature, frozen storage or a different type of plastic. This poses a real problem for manufacturing, shipping and storing of such materials, since plastics are commonly used in single-use bioprocessing bags and storage containers.

### Example 2

### Experimental design and methodology

An Adenoviral preparation comprising Ad26 adenoviruses (as described in [1]) was concentrated and reformulated by ultrafiltration/diafiltration (using a Sartoslice set-up with Pellicon XL Biomax 300 filter, Millipore) to formulations 1, 2 and 3 as defined in Table 1. Formulation 1 is the standard formulation used in example 1, Formulation 2 is the commonly used adenovirus formulation as described in literature [2], and formulation 3 is the commonly used buffer PBS. The three formulations differ in pH, buffering species, and presence and concentration of other excipients.

Subsequently, half of the material was diluted using the appropriate formulation buffer to 1.0×10¹¹ VP/mL (formulation 1) or 1.5×10¹¹ VP/mL (formulations 2 and 3). The other half was spiked with a stock of hydroxypropyl β-cyclodextrin (HBCD) in the appropriate formulation buffer up to a final concentration of 5% (w/w) HBCD, in order to obtain formulations 1, 2 and 3 + HBCD (Table 1). These formulations were also diluted to the same titers using the appropriate formulation buffer. Control samples were taken from all formulations at T=0 before testing the compatibility of the material with bioprocess bags.

**Table 1: Formulations tested in this study.**

| **Formulation** | **Formulation** | **Formulation 1 + HBCD** | **Formulation 2 (Evans *et al*. [2])** | **Formulation 2 (Evans *et al*. [2]) + HBCD** | **Formulation 3 (PBS)** | **Formulation 3 (PBS) + HBCD** |
|---|---|---|---|---|---|---|
| **pH** | 6.5 | 6.5 | 7.4 | 7.4 | 7.4 | 7.4 |
| **buffer** | 20 mM Histidine | 20 mM Histidine | 10 mM Tris and 10 mM Histidine | 10 mM Tris and 10 mM Histidine | 10 mM Na₂HPO₄, 2 mM KH₂PO₄ | 10 mM Na₂HPO₄, 2 mM KH₂PO₄ |
| **cryoprotectant** | 5 % (w/w) Sucrose | 5 % (w/w) HBCD and 5 % (w/w) Sucrose | 5 % (w/w) Sucrose | 5 % (w/w) HBCD and 5 % (w/w) Sucrose | N/A | 5 % (w/w) HBCD |
| **tonicity modifier** | 75 mM NaCl | 75 mM NaCl | 75 mM NaCl | 75 mM NaCl | 137 mM NaCl, 2.7 mM KCl | 137 mM NaCl, 2.7 mM KCl |
| **EtOH (% w/w)** | 0.4 | 0.4 | 0.4 | 0.4 | N/A | N/A |
| **PS-80 (% w/w)** | 0.02 | 0.02 | 0.02 | 0.02 | N/A | N/A |
| **EDTA (mM)** | 0.1 | 0.1 | 0.1 | 0.1 | N/A | N/A |
| **other** | N/A | N/A | 1 mM MgCl2 | 1 mM MgCl2 | N/A | N/A |

To evaluate the plastic compatibility of the Adenovirus formulations 1, 2 and 3 with or without HBCD, 25 mL of each formulation was filled in 50 mL Flexboy^{®} bioprocessing bags (Sartorius Stedim Biotech GmbH), in n=3 per formulation. The bags were incubated at 25 ± 2°C, with time points at 2 or 3 and at 7 or 8 days. At each time point, samples were removed from the bags and stored at ≤ -65°C until sample analysis.

The plastic compatibility of the Adenovirus formulations was analyzed by the following methods, as described above: intrinsic protein fluorescence intensity; hexon protein content by RP-UPLC; and Adenovirus particle concentration by CE. Protein fluorescence and RP-UPLC were performed in triplicate and duplicate, respectively, on all three bags per formulation. CE was performed for two of the three bags, obtaining a single reportable value per bag and time point.

### Results and conclusions

The results presented in Figs. 9-11 clearly show that the Adenovirus formulations 1, 2 and 3 without HBCD are incompatible with plastic during short-term storage at 25°C. Decreases in intrinsic protein fluorescence, hexon content, and viral particle concentration are observed. This indicates that the concentration of (infectious) viral particles and proteins decreases in the formulations, by about a third over the course of one week. As shown in example 1, Ad26 in formulation 1 is stable under these conditions, regarding e.g. protein and particle content, when stored in a compatible primary packaging material such as glass. Formulation 2 is a stable Adenovirus formulation as described in literature [2]. The observed incompatibility is thus specific for the bioprocess bag surface.

The presence of HBCD at 5% (w/w) in formulations 1, 2 or 3 + HBCD has protected the Adenovirus from surface-induced degradation. Figs. 9-11 show that Ad26 in these formulations supplemented with HBCD is compatible with plastic, regarding protein and particle content. These data show that β-cyclodextrins provide a protective effect against plastic surface-induced degradation of viruses that are contained in liquid formulations in bioprocess bags. Importantly, since the effect is observed in all three formulations, despite their differences in pH and excipients, the effect is specific for HBCD and does not depend on the formulation.

### Example 3

### Experimental design and methodology

In the examples above it was demonstrated that, unexpectedly, virus formulations are incompatible with plastic bioprocessing bags, and that the addition of HBCD provides protection against surface-induced degradation from said bags. In order to determine the concentration range in which HBCD protects against surface-induced degradation, the following experiment was performed. An Adenoviral preparation comprising Ad26 adenoviruses (as described in [1]) was concentrated and reformulated by ultrafiltration/diafiltration (using a Sartoslice set-up with Pellicon XL Biomax 300 filter, Millipore) to formulation 2 as defined in Table 1. Formulation 2 is a commonly used adenovirus formulation, as described in literature [2]. Subsequently, the formulation was supplemented with 0, 0.2, 1 or 5% (w/w) hydroxypropyl β-cyclodextrin (HBCD). All formulations were brought to the same titer by diluting with formulation buffer 2. Control samples were taken from all formulations at T=0 before testing the plastic compatibility of the material.

In order to evaluate the compatibility with plastic of the Adenovirus formulations with different HBCD concentrations, 4 mL of each formulation was filled in 5 mL Flexboy^{®} bioprocessing bags (Sartorius Stedim Biotech GmbH), in n=2 per formulation. The bags were incubated at 25 ± 2°C for a compatibility study, with time points at 3 and 8 days. At each time point, samples were removed from the bags and stored at ≤ -65°C until sample analysis.

The plastic compatibility of the Adenovirus formulations was analyzed by the following methods, as described above: intrinsic protein fluorescence intensity; hexon protein content by RP-UPLC; total protein content by absorbance at 280 nm; Adenovirus particle concentration by CE; and potency (infectivity) by QPCR-based Potency Assay (QPA).

### Results and conclusions

Figs. 12 - 16 show the intrinsic protein fluorescence, the total protein concentration by absorbance at 280 nm, the hexon protein content by RP-UPLC, the Adenovirus particle concentration by CE and the potency by QPA, respectively, of the four formulations after 8 days of contact with a plastic bioprocessing bag. At T=0, the protein and particle content of all formulations was the same, but after incubation there is a clear drop in these attributes depending on the HBCD concentration in the formulation. The formulation with 5% HBCD is compatible with the plastic, while the formulations with 0 or 0.2% HBCD have degraded considerable during contact with the plastic: a 20 - 30% drop in viral protein concentration, particle content and potency is observed. The formulation with 1% HBCD shows some improvement in compatibility, but not as much as the formulation with 5% HBCD. HBCD can thus protect against surface-induced degradation already at a concentration of 1%, but its efficacy increases at higher concentrations.

Above 5% and up to at least 30%, full protection is achieved and the formulations are compatible with plastic. The upper limit of the HBCD concentration is thus determined by safety and injectability limits. HBCD is highly soluble in aqueous solutions, and the viscosity and thereby the injectability of the formulation remain within workable ranges up to an HBCD concentration of 50% [3]. HBCD has a good safety and tolerability profile, and no side effects were observed after parenteral administration of up to 24 g of HBCD daily [4]. A solution of 30% HBCD has an osmolarity of ca. 200 - 215 mOsm/L, depending on the molar substitution, and can thus be part of an isotonic formulation. Taking into consideration the factors of compatibility, viscosity and injectability, safety and tolerability, the range in which HBCD can be applied to protect against surface-induced degradation is 1% (w/w) - 30% (w/w).

### REFERENCES

1. Zahn, R., et al., Ad35 and ad26 vaccine vectors induce potent and cross-reactive antibody and T-cell responses to multiple filovirus species. 2012.
2. Evans, R.K., et al., Development of stable liquid formulations for adenovirus-based vaccines. J Pharm Sci, 2004. 93(10): p. 2458-75.
3. Dusautois, C. and S. Neves, Hydroxypropyl Betacyclodextrin: An Enabling Technology for Challenging Pharmaceutical Formulations. Roquette, 2009.
4. Loftsson, T. and M.E. Brewster, Pharmaceutical applications of cyclodextrins: basic science and product development. Journal of pharmacy and pharmacology, 2010. 62(11): p. 1607-1621.

## Claims

1. A method for protecting a virus from surface-induced degradation wherein said virus is contained in a solution in a bag made of a plastic wherein said method comprises the step of adding a β-cyclodextrin to said solution at a concentration between 1% (w/w) to 30% (w/w) wherein said virus is an adenovirus, said β-cyclodextrin is 2-hydroxypropyl-β-cyclodextrin, and the fluid contact layer of said bag is Ethylene Vinyl Acetate.

2. Use of a β-cyclodextrin for protecting a virus from surface-induced degradation wherein said virus is contained in a solution which is contained in a bag made of a plastic and wherein said virus is an adenovirus, said β-cyclodextrin is 2-hydroxypropyl-β-cyclodextrin, and said plastic bag is made of Ethylene Vinyl Acetate.

## Patentansprüche

1. Verfahren zum Schützen eines Virus vor oberflächeninduziertem Abbau, wobei das Virus in einer Lösung in einem Beutel, der aus einem Kunststoff hergestellt ist, enthalten ist, wobei das Verfahren den Schritt eines Hinzufügens eines β-Cyclodextrins zu der Lösung in einer Konzentration zwischen 1 % (Gew./Gew.) bis 30 % (Gew./Gew.) umfasst, wobei das Virus ein Adenovirus ist, das β-Cyclodextrin 2-Hydroxypropyl-β-cyclodextrin ist und die Fluidkontaktschicht des Beutels Ethylenvinylacetat ist.

2. Verwendung eines β-Cyclodextrins zum Schützen eines Virus vor oberflächeninduziertem Abbau, wobei das Virus in einer Lösung enthalten ist, die in einem Beutel, der aus einem Kunststoff hergestellt ist, enthalten ist, wobei das Virus ein Adenovirus ist, wobei das β-Cyclodextrin 2-Hydroxypropyl-β-cyclodextrin ist und der Kunststoffbeutel aus Ethylenvinylacetat hergestellt ist.

## Revendications

1. Procédé destiné à protéger un virus d'une dégradation induite par la surface, dans lequel ledit virus est contenu dans une solution dans un sac constitué d'un plastique, dans lequel ledit procédé comprend l'étape d'ajout d'une β-cyclodextrine à ladite solution à une concentration comprise entre 1 % (p/p) et 30 % (p/p), dans lequel ledit virus est un adénovirus, ladite β-cyclodextrine est 2-hydroxypropyl-β-cyclodextrine, et la couche de contact avec le fluide dudit sac est de l'acétate de vinyle d'éthylène.

2. Utilisation d'une β-cyclodextrine pour protéger un virus d'une dégradation induite par la surface, dans laquelle ledit virus est contenu dans une solution qui est contenue dans un sac constitué d'un plastique et dans lequel, ledit virus est un adénovirus, ladite β-cyclodextrine est 2-hydroxypropyl-β-cyclodextrine, et ledit sac en plastique est constitué d'un acétate de vinyle d'éthylène.
